# EUROPEAN PATENT APPLICATION

(11) **EP 0 788 796 A1**
(43) Date of publication of application: **13.08.1997**
(21) Application number: 96921119.2
(22) Date of filing: 28.06.1996
(51) Int. Cl.: A61K 31/415, C07D 233/80

(54) **URICOSURIC AGENT**

(30) Priority: 30.06.1995 JP 166210/95
(71) Applicant: MOCHIDA PHARMACEUTICAL CO., LTD., Shinjuku-ku Tokyo 160 (JP)
(72) Inventor: KATO, Katsuaki Mochida Pharmaceutical Co., Ltd., Tokyo 160 (JP); OHNISHI, Shuhei Mochida Pharmaceutical Co., Ltd., Tokyo 160 (JP)
(74) Representative: Grünecker, Kinkeldey, Stockmair & Schwanhäusser Anwaltssozietät
(86) International application number: JP9601801
(87) International publication number: WO9702033

(57) **Abstract**

A remedy for hyperuricemia which comprises a hydantoin derivative represented by general formula (I) or a salt thereof as the active ingredient, wherein Q represents cyclohexyl, cycloheptyl, cyclooctyl, 2,5-dimethylthien-3-yl, indan-2-yl or 2,5-dichlorophenyl. The uricosuric agent exerts a potent uricosuric effect on an animal experiment model while sustaining or gradually increasing the urine volume. Further, it has a low toxicity. Thus it is useful in the treatment of hyperuricemia or in the treatment and prevention of gout. It is expected that the effect of the uricosuric agent of sustaining or gradually increasing the urine volume would relieve the load on a patient of taking a large amount of water.

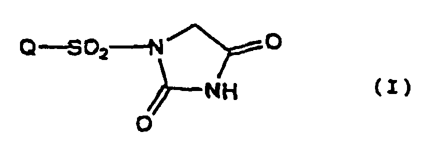

## Description

### TECHNICAL FIELD

This invention relates to novel hydantoin derivatives, as well as hydantoin derivative containing uricosuric agents that are effective in the treatment or prevention/treatment of abnormalities in purine metabolism such as hyperuricemia and gout.

### BACKGROUND ART

For the prevention and treatment of hyperuricemia, gout and the like, there have conventionally been used therapeutics against gouty attacks such as colchicine, analgesic and anti-inflammatory agents such as indomethacin and phenylbutazone, uricosuric agents such as probenecid, benzbromarone and sulfinpyrazone, and inhibitors of uric acid synthesis such as allopurinol. However, these drugs have problems to be solved in terms of limitations in use, side effects and the like as will be described later and it has been desired to develop preventives and therapeutics that are effective against the diseases of interest and which are safe to use.

The prior art related to the compounds of the invention includes the following. Unexamined Published Japanese Patent Application (Kokai) Sho 62-67075, Hei 4-128266 and Hei 3-294270 have disclosures about 1-(2,5-dichlorophenylsulfonyl)hydantoin, 1-(cyclohexylsulfonyl)hydantoin and sulfonylhydantoin derivatives of a general formula having a monocyclic hetero ring group which may be substituted by a straight chained or branched alkyl group having 1 - 8 carbon atoms, as well as their aldose reductase inhibiting action, hypoglycemic action and/or hypolipidemic action.

Such prior art obviously differs from the present invention which aims at treating hyperuricemia and preventing or treating gout and the like.

Unexamined Published Japanese Patent Application (Kokai) Hei 6-211657 discloses to the effect that 1-(3-bromobenzo[b]furan-2-ylsulfonyl)hydantoin shows a uricosuric action but it has no disclosure to the effect that said compound has a mild diuretic action.

Neither prior art reference has a disclosure to the effect that the compounds of the present invention have a uricosuric action.

### DISCLOSURE OF INVENTION

For the problems with the conventional uricosuric agents, reference may be made to "IYAKUHIN YORAN", Fifth Edition, pages 104 - 105 and 1590 - 1593, 1992, Edited by Osaka-fu Byoin Yakuzaishi-kai, published by Yakugyo Jihosha. To be specific, the conventional uricosuric agents listed above can accelerate the excretion of uric acid but, on the other hand, they have the potential to form uric acid stones in renal urinary tubules, which may cause symptoms such as hematuria, renal colic, costalgia, spinalgia, etc. Therefore, general caution to be exercised in prescribing these drugs is to increase the urine volume by water intake and alkalinize urine; thus, those drugs impose undue burden on patients, namely, urinary excretion due to forced water intake.

Among the existing drugs, those of a pyrazolone class are mostly used for nosotropic purposes only on account of the serious side effects they cause and there is a problem in that they are contraindicated in persons who are sensitive to the compounds of a pyrazolone class.

Furthermore, a case for a serious side effect, i.e., hypoplastic anemia, has been reported with allopurinol which is a uric acid synthesis inhibitor of common use today ("IYAKUHIN NO FUKUSAYO", Annual Report 1990, pages 115 - 118, 1991, Chugai Igakusha).

Generally speaking, the development of compounds as medicines must take the potency of their pharmacological activity into consideration but there are many other problems that should also be dealt with from various viewpoints. Specific examples would include safety, the solubility of the active drug, the stability of the preparation and its bioavailability and, among other things, safety considerations require not only low lethal dose but various parameters such as the presence or absence of teratogenicity, carcinogenicity and enzyme induction must also be reviewed with utmost care and these problems must be overcome by all means before the compounds are qualified as medicines.

Under the circumstances, it is required to develop a drug that is not merely potent in the ability to remedy hyperuricemia but which can also be used safely and conveniently without causing the burden of water intake, thus featuring high safety and good benefit from a viewpoint of pharmaceutical formulation. The purpose of the present invention is to meet at least one of these requirements.

The present inventors made intensive studies with a view to solving the problems of the conventional uricosuric agents and found, as a result, that hydantoin derivatives of the formula (I) had a potent uricosuric action while having the ability to maintain or increase mildly the urine volume; the present invention has been accomplished on the basis of this finding.

A first aspect of the invention relates to a therapeutic agent of hyperuricemia characterized by containing a hydantoin derivative of the general formula (I): 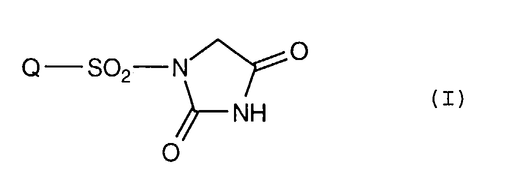 (where Q denotes a cyclohexyl group, a cycloheptyl group, a cyclooctyl group, a 2,5-dimethylthien-3-yl group, an indan-2-yl group or a 2,5-dichlorophenyl group) or a salt thereof as an active ingredient. Stated specifically, the invention relates to a therapeutic agent of hyperuricemia wherein Q in the general formula (I) is a cyclohexyl group, a therapeutic agent of hyperuricemia wherein Q in the general formula (I) is a cycloheptyl group, a therapeutic agent of hyperuricemia wherein Q in the general formula (I) is a cyclooctyl group, a therapeutic agent of hyperuricemia wherein Q in the general formula (I) is a 2,5-dimethylthien-3-yl group, a therapeutic agent of hyperuricemia wherein Q in the general formula (I) is an indan-2-yl group, or a therapeutic agent of hyperuricemia wherein Q in the general formula (I) is a 2,5-dichlorophenyl group.

In addition, a second aspect of the invention relates to an agent for preventing and treating gout which is characterized by containing a hydantoin derivative of the general formula (I) (where Q has the same definition as given above) or a salt thereof as an active ingredient. Stated specifically, the invention relates to an agent for preventing and treating gout wherein Q in the general formula (I) is a cyclohexyl group, an agent for preventing and treating gout wherein Q in the general formula (I) is a cycloheptyl group, an agent for preventing and treating gout wherein Q in the general formula (I) is a cyclooctyl group, an agent for preventing and treating gout wherein Q in the general formula (I) is a 2,5-dimethylthien-3-yl group, an agent for preventing and treating gout wherein Q in the general formula (I) is an indan-2-yl group, or an agent for preventing and treating gout wherein Q in the general formula (I) is a 2,5-dichlorophenyl group.

In addition, a third aspect of the invention relates to a uricosuric agent characterized by containing a hydantoin derivative of the general formula (I) (where Q has the same definition as given above) or a salt thereof as an active ingredient. Stated specifically, the invention relates to a uricosuric agent wherein Q in the general formula (I) is a cyclohexyl group, a uricosuric agent wherein Q in the general formula (I) is a cycloheptyl group, a uricosuric agent wherein Q in the general formula (I) is a cyclooctyl group, a uricosuric agent wherein Q in the general formula (I) is a 2,5-dimethylthien-3-yl group, a uricosuric agent wherein Q in the general formula (I) is an indan-2-yl group, or a uricosuric agent wherein Q in the general formula (I) is a 2,5-dichlorophenyl group.

In addition, a fourth aspect of the invention relates to a hydantoin derivative of the general formula (I'): 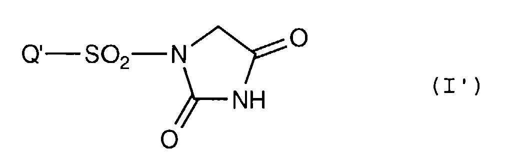 (where Q' denotes a cycloheptyl group, a cyclooctyl group, a 2,5-dimethylthien-3-yl group or an indan-2-yl group) or a salt thereof. Stated specifically, the invention relates to a hydantoin derivative wherein Q' in the general formula (I') is a cycloheptyl group or a salt thereof, a hydantoin derivative wherein Q' in the general formula (I') is a cyclooctyl group or a salt thereof, a hydantoin derivative wherein Q' in the general formula (I') is a 2,5-dimethylthien-3-yl group or a salt thereof, or a hydantoin derivative wherein Q' in the general formula (I') is an indan-2-yl group or a salt thereof.

Salts of the compounds (I') to be used in the invention include, for example, alkali metal salts such as sodium salt and potassium salt, alkaline earth metal salts such as magnesium salt and calcium salt, or inorganic bases such as ammonium salt, organic base salts such as benzylamine salt and diethylamine salt, and amino acid salts such as arginine salt and lysine salt.

In addition, a fifth aspect of the invention relates to a process for producing a hydantoin derivative of the general formula (I'): 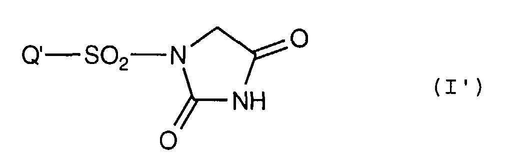 (where Q' denotes a cycloheptyl group, a cyclooctyl group, a 2,5-dimethylthien-3-yl group or an indan-2-yl group) or a salt thereof, comprising the step of reacting a sulfonyl glycine derivative of the general formula (II):

Q'-SO₂NHCH₂COR (II)

(where Q' denotes a cycloheptyl group, a cyclooctyl group, a 2,5-dimethylthien-3-yl group or an indan-2-yl group, and R denotes a hydroxyl group or an alkoxy group of 1 - 4 carbon atoms such as methoxy or ethoxy) with a thiocyanate salt to form a thiohydantoin derivative of the general formula (III): 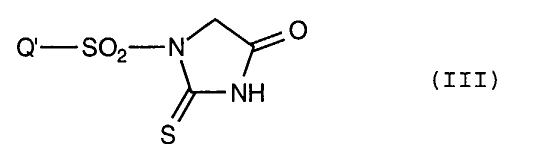 (where Q' has the same meaning as defined above) and the step of oxidizing or hydrolyzing said derivative.

Further, a sixth aspect of the invention relates to a sulfonylglycine derivative of the general formula (II):

Q'-SO₂NHCH₂COR (II)

(where Q' denotes a cycloheptyl group, a cyclooctyl group, a 2,5-dimethylthien-3-yl group or an indan-2-yl group, and R denotes a hydroxyl group or an alkoxy group of 1 - 4 carbon atoms such as methoxy or ethoxy). The sixth aspect of the invention is useful as an intermediate for the production of the hydantoin derivative according to the fourth aspect of the invention which is represented by the general formula (I').

Further, a seventh aspect of the invention relates to the use of a hydantoin derivative of the general formula (I) (where Q has the same definition as given above) or a salt thereof for the production of drugs as therapeutics of hyperuricemia, therapeutics for gout and/or uricosuric agents. Stated specifically, the invention relates to the use of a hydantoin derivative wherein Q in the general formula (I) is a cyclohexyl group or a salt thereof, the use of a hydantoin derivative wherein Q in the general formula (I) is a cycloheptyl group or a salt thereof, the use of a hydantoin derivative wherein Q in the general formula (I) is a cyclooctyl group or a salt thereof, the use of a hydantoin derivative wherein Q in the general formula (I) is a 2,5-dimethylthien-3-yl group or a salt thereof, the use of a hydantoin derivative wherein Q in the general formula (I) is an indan-2-yl group or a salt thereof, or the use of a hydantoin derivative wherein Q in the general formula (I) is a 2,5-dichlorophenyl group or a salt thereof.

Further, an eighth aspect of the invention relates to a method of treating hyperuricemia and/or a method of preventing and treating gout by an effective amount of a hydantoin derivative of the general formula (I) (where Q has the same definition as given above) or a salt thereof. Stated specifically, the invention relates to a method of treating hyperuricemia and/or a method of preventing and treating gout, wherein Q in the general formula (I) is a cyclohexyl group, a method of treating hyperuricemia and/or a method of preventing and treating gout, wherein Q in the general formula (I) is a cycloheptyl group, a method of treating hyperuricemia and/or a method of preventing and treating gout, wherein Q in the general formula (I) is a cyclooctyl group, a method of treating hyperuricemia and/or a method of preventing and treating gout, wherein Q in the general formula (I) is a 2,5-dimethylthien-3-yl group, a method of treating hyperuricemia and/or a method of preventing and treating gout, wherein Q in the general formula (I) is an indan-2-yl group, or a method of treating hyperuricemia and/or a method of preventing and treating gout, wherein Q in the general formula (I) is a 2,5-dichlorophenyl group.

Further, a ninth aspect of the invention relates to a method of excreting uric acid by an effective amount of a hydantoin derivative of the general formula (I) (where Q has the same definition as given above) or a salt thereof. Stated specifically, the invention relates to a method of excreting uric acid wherein Q in the general formula (I) is a cyclohexyl group, a method of excreting uric acid wherein Q in the general formula (I) is a cycloheptyl group, a method of excreting uric acid wherein Q in the general formula (I) is a cyclooctyl group, a method of excreting uric acid wherein Q in the general formula (I) is a 2,5-dimethylthien-3-yl group, a method of excreting uric acid wherein Q in the general formula (I) is an indan-2-yl group, or a method of excreting uric acid wherein Q in the general formula (I) is a 2,5-dichlorophenyl group.

In the general formula (I), Q is a cyclohexyl group, a cycloheptyl group, a cyclooctyl group, a 2,5-dimethylthien-3-yl group, an indan-2-yl group or a 2,5-dichlorophenyl group; preferred is a cyclohexyl group, a cycloheptyl group, a 2,5-dimethylthien-3-yl group or an indan-2-yl group, and more preferred is a cycloheptyl group.

In the general formulas (I'), (II) and (III), Q' is a cycloheptyl group, a cyclooctyl group, a 2,5-dimethylthien-3-yl group or an indan-2-yl group, with the cycloheptyl group being preferred.

The method of synthesis of 1-(2,5-dichlorophenylsulfonyl)hydantoin is disclosed in Unexamined Published Japanese Patent Application (Kokai) Sho 62-67075, and that of 1-(cyclohexylsulfonyl)hydantoin is disclosed in Unexamined Published Japanese Patent Application (Kokai) Hei 4-128266; these methods can also be used to synthesize 1-(cycloheptylsulfonyl)hydantoin, 1-(cyclooctylsulfonyl) hydantoin, 1-(2,5-dimethylthien-3-ylsulfonyl)hydantoin and 1-(indan-2-ylsulfonyl)hydantoin.

### BEST MODE FOR CARRYING OUT THE INVENTION

The methods will now be described below in detail.

The synthesis of sulfonyl halides represented by Q-SO₂-X (Q has the same meaning as in the general formula (I), and X denotes a halogen atom) is described in detail in SHIN-JIKKEN KAGAKU KOZA (14), YUKI KAGOBUTSU NO GOSBI TO HANNO (III), pages 1784 - 1792 (edited by The Chemical Society of Japan, published by Maruzen Kabushiki Kaisha, 1978) and in the present invention, the following methods may be employed.

In one example, a compound of the formula Q-X (Q has the same meaning as in the general formula (I), and X is a halogen atom) is rendered an organolithium compound with the aid of a suitable base such as n-butyl lithium or metallic lithium, or converted to a Grignard reagent with the aid of metallic magnesium, followed by reaction with sulfur dioxide or a thionyl halide; subsequently, the reaction product is treated with a suitable oxidizer such as chlorine in the presence of halide ions to yield a sulfonyl halide of the formula Q-SO₂-X (Q and X have the same meanings as defined above). Alternatively, an organolithium compound or a Grignard reagent is reacted with a sulfuryl halide to yield a sulfonyl halide directly and this is also an effective method.

According to another common method, Q-SY (Q has the same meaning as defined above, and Y is H, acetyl, benzyl or SO₃Na) or Q-S-S-Q (disulfide; Q has the same meaning as defined above) which can be easily derived from a suitable starting compound such as a cycloalkene, indene or Q-X (Q and X have the same meanings as defined above) is treated with a suitable oxidizer such as chlorine gas, bromine or potassium chlorate (KClO₃) in hydrochloric acid, preferably using potassium chlorate in hydrochloric acid, to yield a sulfonyl halide of the formula Q-SO₂-X (Q and X have the same meanings as defined above).

In the case where Q is aromatic, an aromatic compound Q-H may be reacted with a suitable halosulfonating agent such as a halosulfonic acid in the presence or absence of a pentahalophosphoric acid in the absence of any solvent or in the presence of a suitable solvent such as methylene chloride, thereby yielding a sulfonyl halide of the formula Q-SO₂-X.

Subsequently, the sulfonyl halide is reacted with a glycine derivative represented by H₂NCH₂CO-Z (where Z denotes a hydroxyl group, an alkoxy group of 1 - 4 carbon atoms such as methoxy or ethoxy, or an amino group which may be substituted by an alkoxycarbonyl group of 2 - 5 carbon atoms such as methoxycarbonyl or ethoxycarbonyl) to synthesize an N-sulfonylglycine derivative. The treatment of the product varies with the kind of the glycine derivative and speaking of the sulfonyl glycine derivative of interest,
1) if Z is an amino group, reaction with an alkyl haloformate is performed in the presence of a suitable base such as sodium hydride,
2) if Z is an amino group substituted by an alkoxycarbonyl group, heat treatment is performed in the presence or absence of a base such as sodium hydride, and
3) if Z is a hydroxyl group or an alkoxy group, treatment with a thiocyanate salt in the presence of an acid anhydride such as acetic anhydride is performed to effect cyclization to a corresponding thiohydantoin derivative, followed by treatment with a suitable oxidizer such as hydrogen peroxide in acetic acid, nitric acid or iodine monochloride, thereby preparing the corresponding hydantoin derivatives in the respective cases.

The present invention will now be described in detail with reference to experiment cases.

### Experiment Case 1

### Uric Acid Excreting and Diuretic Actions in Rats

Three female Wistar rats (4-week old) in each group were administered orally with 300 or 1000 mg/kg of a test compound in a volume of 10 ml/kg as suspended in an aqueous solution of 0.5% carboxymethylcellulose sodium or 5% gum arabic; immediately thereafter, the animals were loaded orally with 25 ml/kg of physiological saline and the urine was collected up to 5 h after the administration of the test compound. The collected urine was immediately measured quantitatively and the urinary concentration of uric acid was measured by a urikase method in accordance with the procedure of Kabasakalian et al. [Kabasakalian, P. et al.: Clin Chem., 19, 522-524 (1973)] and the urinary excretion of uric acid was calculated. As control drugs, probenecid (300 or 1000 mg/kg) and furosemide (30 mg/kg) were used. Data on the individual groups are shown in Table 1 in relative values, with 100 being assigned to each of the volume of urine and the urinary excretion of uric acid in a control group which was administered with an aqueous solution of 0.5% carboxymethylcellulose sodium or 5% gum arabic in place of the solution of a test compound.

**Table 1**

| Drug | Dose (mg/kg) | Urine Volume (%) | Urinary excretion of uric acid (%) |
|---|---|---|---|
| Control | - | 100 | 100 |
| Compound 1 | 300 | 96 | 116 |
| | 1000 | 124 | 155 |
| Compound 2 | 300 | 159 | 152 |
| | 1000 | 170 | 256 |
| Compound 3 | 300 | 109 | 103 |
| | 1000 | 95 | 245 |
| Compound 4 | 300 | 111 | 104 |
| | 1000 | 207 | 145 |
| Compound 5 | 300 | 135 | 102 |
| | 1000 | 144 | 144 |
| Compound 6 | 300 | 151 | 96 |
| | 1000 | 223 | 151 |
| Probenecid | 300 | 60 | 78 |
| | 1000 | 20 | 167 |
| Furosemide | 30 | 215 | 106 |

By the administration of the compounds of the invention, there were observed marked increases in the urinary excretion of uric acid in the animals and the potency of the compounds tested was comparable to or higher than that of a conventional drug, probenecid. As for the urine volume, probenecid significantly decreased the urine volume at the effective doses for uric acid excretion; in contrast, the uricosuric agents of the invention did not cause a decrease in the urine volume and, what is more, a marked increase in the urine volume occurred depending on the dose. With furosemide, an increase in the urine volume was observed but no increase was observed in the uric acid excretion.

Test compounds 1 - 6 used in the experiment are identified below.
- Compound 1:: 1-(cyclohexylsulfonyl)hydantoin
- Compound 2:: 1-(cycloheptylsulfonyl)hydantoin
- Compound 3:: 1-(cyclooctylsulfonyl)hydantoin
- Compound 4:: 1-(2,5-dimethylthien-3-ylsulfonyl)hydantoin
- Compound 5:: 1-(indan-2-ylsulfonyl)hydantoin
- Compound 6:: 1-(2,5-dichlorophenylsulfonyl)hydantoin

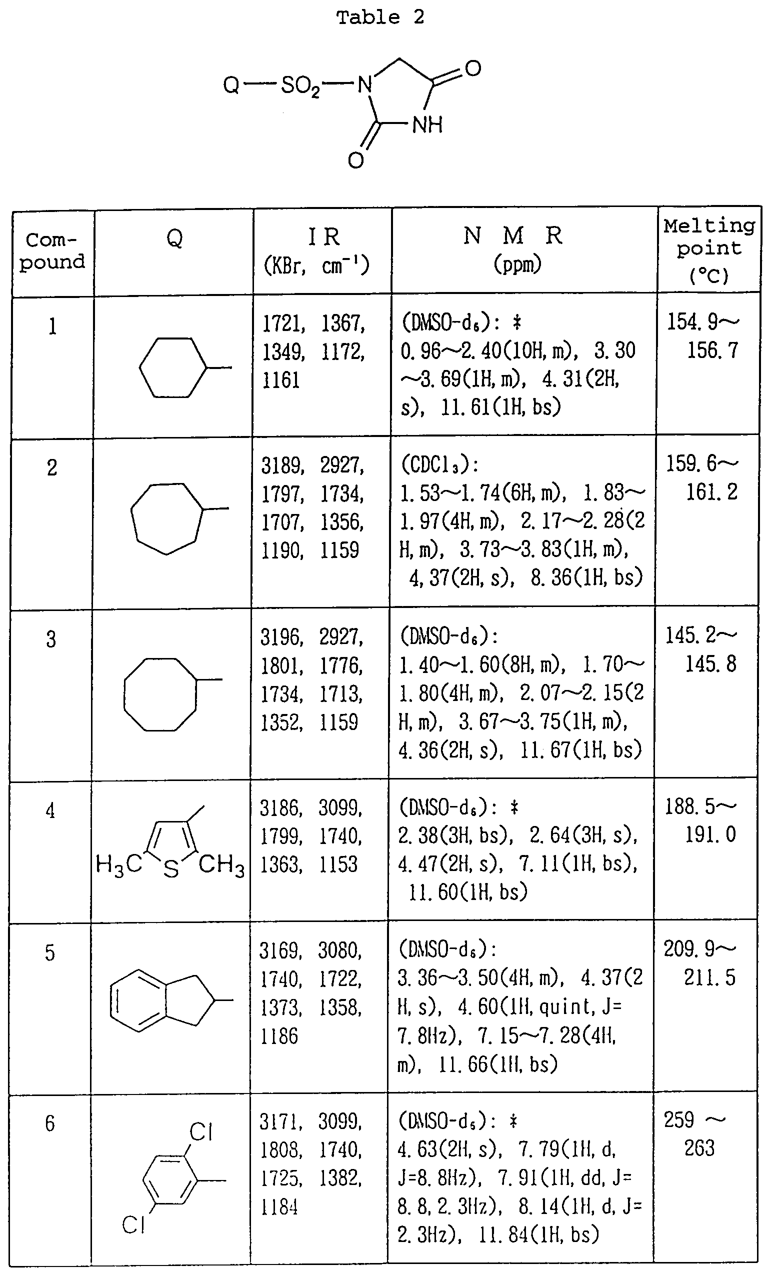

### Experiment Case 2

### Acute Toxicity Test

Three female Wistar rats (4-week old) in each group were administered orally with 100 - 1000 mg/kg of a test compound in a volume of 10 ml/kg as suspended in an aqueous solution of 0.5% carboxymethylcellulose sodium or 5% gum arabic and were subsequently observed over 24 h. The results are shown in Table 3. Compounds 1 - 6 were as identified above.

**Table 3**

| Drug | Acute toxicity (Minimal dose causing death: mg/kg) |
|---|---|
| Compound 1 | >1000 (no death case at 1000) |
| Compound 2 | >1000 (no death case at 1000) |
| Compound 3 | >1000 (no death case at 1000) |
| Compound 4 | >1000 (no death case at 1000) |
| Compound 5 | >1000 (no death case at 1000) |
| Compound 6 | >1000 (no death case at 1000) |

With the compounds of the invention, side effects and other abnormalities were not observed at all at the doses at which they exhibited the pharmacological action.

A test was also conducted to administer 100 - 300 mg/kg of the compounds of the invention to rats for 4 consecutive days but there was observed no enzyme induction which would occur in hydantoin derivatives such as phenytoin. The compounds of the invention showed no toxicity in tests using other toxicological indices.

From these results, it is concluded that the compounds of the invention are drugs that exhibit a potent uricosuric action in animals while maintaining or mildly increasing the urine volume; at the same time, and have low toxicity, thus they are qualified as medicines particularly suited to the treatment of hyperuricemia and to the treatment and prevention of gout.

Gout is a disease that occurs when the serum level of uric acid exceeds a certain value to cause crystallization of sodium urate, which is deposited in joint cavities and kidneys. Therefore, treatment of hyperuricemia can result in the prevention of gout.

Salts of the compounds of the general formula (I) which are to be used in the invention include pharmaceutically acceptable salts such as, for example, alkali metal salts such as sodium salt and potassium salt, alkaline earth metal salts such as magnesium salt and calcium salt or inorganic bases such as ammonium salt, organic base salts such as benzylamine salt and diethylamine salt, and amino acid salts such as arginine salt and lysine salt.

The uricosuric agents of the invention are such that at least one of the hydantoin derivatives of the general formula (I) or salts thereof is combined with pharmaceutically acceptable vehicles or solvents, such as distilled water, vegetable oils and even innocuous organic solvents and solubilization aids (e.g., ethanol, acetic acid, glycerin and propylene glycol), excipients, binders, lubricants, colorants, flavors, emulsifiers and suspending agents, in appropriate manners to formulate the following dosage forms.

Specific examples of such dosage forms include oral preparations such as tablets, capsules, granules, subtilized granules, powders, suppositories and syrups, as well as inhalants, ointments, aqueous or nonaqueous injections, emulsified or suspended injections, and solid injections which are dissolved, emulsified or suspended just prior to use, and these dosage forms may be administered to patients irrespective of whether the route is oral or parenteral (e.g. rectal administration, transdermal absorption, absorption through the mucous membrane, intravenous administration, intramuscular administration or subcutaneous administration). In the case of oral preparations, injections, suppositories and inhalants, their daily dose is 1 mg - 5000 mg, preferably 10 - 500 mg as calculated for the hydantoin derivatives of interest and in the case of ointments, suitable amounts may be applied as 1 - 10% ointments; in either case, the dose can be increased or decreased depending upon the condition of the patient; if desired, the entire portion may be administered at a time or divided in 2 - 6 portions or sustained administration is also possible such as by dripping.

### EXAMPLES

### Example 1 Synthesis of 1-(cyclohexylsulfonyl)hydantoin (Compound 1)

### 〈Step 1〉 Synthesis of cyclohexylsulfonyl chloride

Potassium chlorate (272 g) was added to a mixture of cyclohexane thiol (199 g) and 8 N HCl (3 L) as it was held at a temperature of 10 - 20°C. After 3 h stirring, ether (700 mL) was added to the reaction solution for extraction and the ethereal layer was collected. The aqueous layer was subjected to three additional extractions with ether (700 mL) and the ethereal layers were combined, washed with water and dried with anhydrous sodium sulfate and, thereafter, the solvent was distilled off under reduced pressure. The residue was distilled under reduced pressure to yield the titled compound (206 g) as an oil. b.p. 113 - 115°C (14 mmHg)
IR spectrum (neat; cm⁻¹)
   2945, 2941, 1453, 1373, 1161
NMR spectrum (CDCl₃; ppm)*
   1.23 - 2.50 (10H, m)
   3.39 - 3.65 (1H, m)

### 〈Step 2〉 Synthesis of N-(cyclohexylsulfonyl)glycine

The compound (200 g) obtained in step 1 was dissolved in methylene chloride (3 L) and, following the addition of glycine ethyl ester hydrochloride (306.6 g), the mixture was stirred under cooling with ice water while triethylamine (321 mL) was added dropwise. After 3.5 h stirring at room temperature, the reaction solution was washed successively with water, 1 N HCl and saturated brine, dried with anhydrous sodium sulfate, with the solvent being subsequently distilled off under reduced pressure.

The resulting crude glycine ethyl ester was dissolved in methanol (2870 mL) and the solution was stirred at room temperature for 45 min. while an aqueous solution of 3 N sodium hydroxide (570 mL) was added dropwise under cooling with ice water. Methanol was distilled off the reaction solution under reduced pressure and a suitable amount of water was added to the residue, which was adjusted to pH of 1 with conc. HCl and subjected to extraction with ethyl acetate (1200 mL). The organic layer was washed successively with water and saturated brine and dried with anhydrous sodium sulfate and, thereafter, the solvent was distilled off under reduced pressure. Hexane was added to the residue for crystallization, which were collected by filtration to yield the titled compound (87.5 g). m.p. 96.0 - 100.9°C
IR spectrum (KBr; cm⁻¹)
   3308, 1714, 1319, 1147, 1126
NMR spectrum (DMSO-d₆; ppm)*
   1.18 - 2.06 (10H, m)
   2.64 - 3.19 (1H, m)
   3.69 (2H, d, J=6.0 Hz)
   7.33 (1H, t, J=6.0 Hz)

### 〈Step 3〉 Synthesis of 1-(cyclohexylsulfonyl)thiohydantoin

To a mixture of the compound (23.5 g) obtained in step 2 and acetic anhydride (50 mL), there was added ammonium thiocyanate (17.8 g) and pyridine (22.3 mL) was added dropwise. After stirring at 40 - 50°C for 30 min., the reaction solution was poured into water (200 mL) and two extractions were conducted with ethyl acetate (500 mL). The organic layers were combined, washed successively with 1 N HCl, a saturated aqueous solution of sodium hydrogencarbonate and saturated brine and dried with anhydrous sodium sulfate and, thereafter, the solvent was distilled off under reduced pressure. The residue was purified by silica gel column chromatography (eluting solvent: chloroform) and the resulting product was crystallized with ether-hexane, the crystals being collected by filtration to yield the titled compound (17.3 g).
IR Spectrum (KBr; cm⁻¹)
   1791, 1757, 1735, 1453, 1353, 1236, 1169
NMR Spectrum (DMSO-d₆; ppm)*
   1.24 - 2.23 (10H, m)
   3.90 - 4.32 (1H, m)
   4.50 (2H, s)
   12.70 (1H, bs)

### 〈Step 4〉 Synthesis of 1-(cyclohexylsulfonyl)hydantoin

The compound (52 g) obtained in step 3 was suspended in acetic acid (520 mL) and 30% aqueous hydrogenperoxide (68 mL) was added dropwise slowly at 60°C. After 1 h stirring, the insoluble material was filtered off and poured into water (3 L), with the precipitating crystals being collected by filtration and dried to yield the titled compound (25 g). The analytical data are shown in Table 2 under "Compound 1".

### Example 2 Synthesis of 1-(cycloheptylsulfonyl)hydantoin (Compound 2)

### 〈Step 1〉 Synthesis of acetylthiocycloheptane

Thioacetic acid (17 mL) was added to cycloheptene (25 mL) under cooling with ice water and the mixture was stirred at room temperature for 10 h. The reaction solution was diluted with ether (150 mL), washed with a saturated aqueous solution of sodium hydrogencarbonate and saturated brine and dried with anhydrous sodium sulfate; thereafter, the solvent was distilled off under reduced pressure to yield the titled compound (37 g) as an oil.
IR Spectrum (neat; cm⁻¹)
   2927, 2854, 1689, 1458, 1140, 1113, 953, 635
NMR Spectrum (CDCl₃; ppm)*
   1.32 - 2.00 (12H, m)
   2.28 (3H, s)
   3.39 - 3.77 (1H, m)

### 〈Step 2〉 Synthesis of N-(cycloheptylsulfonyl)glycine

To a mixture of the compound (37 g) obtained in step 1 and 8 N HCl (360 mL), there was added dropwise an aqueous solution of 0.68 M potassium chlorate (350 mL) at 20°C or below under cooling with ice water. After stirring at room temperature for 10 h, the reaction solution was subjected to two extractions with ether (500 mL). The ethereal layers were combined and after washing with water and saturated brine, they were dried with anhydrous sodium sulfate and had the solvent distilled off under reduced pressure.

The resulting crude cycloheptylsulfonyl chloride was added dropwise to a suspension of glycine ethyl ester hydrochloride (81 g) and triethylamine (80 mL) in methylene chloride (500 mL) under cooling with ice water. After stirring at room temperature for 2 h, the reaction solution was washed successively with water, 1 N HCl and saturated brine and dried with anhydrous sodium sulfate; thereafter, the solvent was distilled off under reduced pressure.

To the resulting crude glycine ethyl ester as dissolved in ethanol (100 mL), there was added dropwise an aqueous solution of 1.5 N sodium hydroxide (50 mL) under cooling with ice water and the mixture was stirred at room temperature for 1 h. Ethanol was distilled off under reduced pressure and water (100 mL) was added to the residue; the solution was adjusted to pH of 1 with conc. HCl and subjected to three extractions with ethyl acetate (100 mL). The organic layers were combined, washed successively with water and saturated brine and dried with anhydrous sodium sulfate; thereafter, the solvent was distilled off under reduced pressure. Hexane was added to the residue and the resulting crystals were collected by filtration to yield the titled compound (15 g).
m.p. 83.3 - 88.2°C
IR Spectrum (KBr; cm⁻¹)
   3336, 2929, 2858, 1722, 1429, 1313, 1257, 1140
NMR Spectrum (CDCl₃; ppm)*
   1.20 - 2.46 (12H, m)
   2.82 - 3.24 (1H, m)
   3.98 (2H, d, J=5.6 Hz)
   5.03 (1H, t, J=5.6 Hz)

### 〈Step 3〉 Synthesis of 1-(cycloheptylsulfonyl)thiohydantoin

To a mixture of the compound (92.5 g) obtained in step 2 and ammonium thiocyanate (90 g), there was added pyridine (96 mL) and acetic anhydride (185 mL) was added slowly under cooling with water. After stirring at 50 - 60°C for 3 h, the reaction solution was poured into 1 N HCl (1.5 L) and the precipitating crystals were collected by filtration. The crystals were dissolved in ethyl acetate (1 L) and washed with water and saturated brine; thereafter, the solution was dried with anhydrous sodium sulfate and the solvent was distilled off under reduced pressure. The residue was crystallized with ether-hexane and the crystals were collected by filtration to yield the titled compound (78 g).
m.p. 132.1 - 136.9°C
IR Spectrum (KBr; cm⁻¹)
   3147, 2927, 1761, 1452, 1352, 1236, 1163, 1078
NMR Spectrum (CDCl₃; ppm)
   1.50 - 1.70 (6H, m)
   1.85 - 1.97 (4H, m)
   2.16 - 2.24 (2H, m)
   4.50 (2H, s)
   4.39 - 4.50 (1H, m)
   8.84 (1H, bs)

### 〈Step 4〉 Synthesis of 1-(cycloheptylsulfonyl)hydantoin

The compound (78 g) obtained in step 3 was dissolved in acetic acid (800 mL) and 30% aqueous hydrogen peroxide (96 mL) was added dropwise slowly at 60°C. After 1 h stirring, the insoluble material was filtered off and the filtrate was poured into water (5 L) and the precipitating crystals were collected by filtration. The crystals were dissolved in ethyl acetate (1 L) and washed successively with water and saturated brine. The ethyl acetate layer was dried with anhydrous sodium sulfate and, thereafter, the solvent was distilled off under reduced pressure. The residue was purified by silica gel column chromatography (eluting solvent: ethyl acetate/hexane = 1/1) and, thereafter, the crude product was crystallized with ether to yield the titled compound (36 g). The analytical data are shown in Table 2 under "Compound 2".

### Example 3 Synthesis of 1-(cyclooctylsulfonyl)hydantoin (Compound 3)

### 〈Step 1〉 Synthesis of acetylthiocyclooctane

Cyclooctene (20 g) was dissolved in anhydrous hexane (100 mL) and 2,2'-azobisisobutyronitrile (1.5 g) and thioacetic acid (20 mL) were added, followed by refluxing for 5 h under an argon atmosphere. After being left to cool, the reaction solution was poured into water (200 mL) and extraction was conducted with ether (200 mL). The ethereal layer was washed successively with a saturated aqueous solution of sodium hydrogencarbonate and saturated brine and dried with anhydrous sodium sulfate; thereafter, the solvent was distilled off under reduced pressure. The residue was purified by silica gel column chromatography (eluting solvent: hexane) to yield the titled compound (28 g) as an oil.
IR Spectrum (neat; cm⁻¹)
   2924, 2852, 1689, 1473, 1446, 1352, 1140, 1113
NMR Spectrum (CDCl₃; ppm)
   1.51 - 1.75 (12H, m)
   1.89 - 1.99 (2H, m)
   2.29 (3H, s)
   3.62 - 3.74 (1H, m)

### 〈Step 2〉 Synthesis of N-(cyclooctylsulfonyl)glycine

To a mixture of the compound (27 g) obtained in step 1 and 8 N HCl (245 mL), potassium chlorate (23 g) was added in small portions at 25°C or below under cooling with ice water. After stirring at room temperature for 2 h, the reaction solution was subjected to two extractions with 300 mL of ether. The ethereal layers were combined, washed with water and saturated brine and thereafter dried with anhydrous sodium sulfate and had the solvent distilled off under reduced pressure.

The resulting crude cyclooctylsulfonyl chloride was dissolved in methylene chloride (300 mL) and glycine ethyl ether hydrochloride (56 g) was added and with continued stirring under cooling with ice water, triethylamine (56 mL) was added dropwise. After stirring at room temperature for 1 h, the reaction solution was washed successively with water, 1 N HCl and saturated brine and dried with sodium anhydrous sulfate; thereafter, the solvent was distilled off under reduced pressure.

The resulting crude glycine ethyl ester was dissolved in ethanol (200 mL) and an aqueous solution of 1.5 N sodium hydroxide (100 mL) was added dropwise under cooling with ice water and the mixture was stirred at room temperature for 1 h. Ethanol was distilled off the reaction solution under reduced pressure and water (100 mL) was added to the residue; the solution was adjusted to pH of 1 with conc. HCl and subjected to three extractions with ethyl acetate (100 mL). The organic layers were combined and washed successively with water and saturated brine and dried with anhydrous sodium sulfate; thereafter, the solvent was distilled off under reduced pressure. Hexane was added to the residue for crystallization, which were collected to yield the titled compound (15.5 g).
m.p. 105.5 - 107.1°C
IR Spectrum (KBr; cm⁻¹)
   3334, 3203, 2926, 1767, 1755, 1311, 1200, 1143
NMR Spectrum (DMSO-d₆; ppm)
   1.39 - 1.71 (12H, m)
   2.05 - 2.12 (2H, m)
   3.06 - 3.12 (1H, m)
   3.71 (2H, d, J=5.9 Hz)
   7.45 (1H, t, J=5.9 Hz)
   12.71 (1H, bs)

### 〈Step 3〉 Synthesis of 1-(cyclooctylsulfonyl)thiohydantoin

The compound (14 g) obtained in step 2 was treated by the same method as step 3 in Example 1 to yield the titled compound (10.9 g).
m.p. 137.4 - 139.0°C
IR Spectrum (KBr; cm⁻¹)
   3151, 2923, 2854, 1790, 1761, 1751, 1736, 1446, 1346
NMR Spectrum (DMSO-d₆; ppm)
   1.40 - 1.60 (8H, m)
   1.70 - 1.80 (4H, m)
   2.05 - 2.14 (2H, m)
   4.51 - 4.54 (1H, m)
   4.54 (2H, s)
   12.77 (1H, bs)

### 〈Step 4〉 Synthesis of 1-(cyclooctylsulfonyl)hydantoin

The compound (10 g) obtained in step 3 was treated by the same method as step 4 in Example 2 to yield the titled compound (5.7 g). The analytical data are shown in Table 2 under "Compound 3"

### Example 4 Synthesis of 1-(2,5-dimethylthien-3-ylsulfonyl)hydantoin (Compound 4)

### 〈Step 1〉 Synthesis of 2,5-dimethylthien-3-ylsulfonyl chloride

Phosphorus pentachloride (28 g) was suspended in methylene chloride (50 mL) and chlorosulfonic acid (21 g) was added dropwise slowly under cooling with ice water. After 30 min. stirring, a solution of 2,5-dimethylthiophene (10 g) in methylene chloride (25 mL) was added dropwise slowly. After 2 h stirring, the reaction solution was poured into water (300 mL) and the methylene chloride layer was separated. The aqueous layer was subjected to further extraction with methylene chloride (100 mL). The methylene chloride layers were combined, washed with water twice, then washed with saturated brine once and, thereafter, dried with anhydrous magnesium sulfate and had the solvent distilled off under reduced pressure. The residue was purified by silica gel column chromatography (eluting solvent: ethyl acetate/hexane = 3/97) to yield the titled compound (15 g) as an oil.
IR Spectrum (neat; cm⁻¹)
   2926, 2862, 1487, 1367, 1194, 631
NMR Spectrum (CDCl₃; ppm)*
   2.44 (3H, bs)
   2.73 (3H, s)
   7.06 (1H, bs)

### 〈Step 2〉 Synthesis of N-(2,5-dimethylthien-3-ylsulfonyl)glycine

Glycine (6.4 g) and anhydrous potassium carbonate (11.8 g) were dissolved in water (43 mL); to the solution, the compound (15 g) obtained in step 1 was added and the mixture was stirred at 70°C for 3 h. After cooling, the reaction solution was adjusted to pH of 1 with conc. HCl and subjected to three extractions with ethyl acetate (150 mL). The ethyl acetate layers were dried with anhydrous magnesium sulfate and the solvent was distilled off under reduced pressure. Hexane was added to the residue for crystallization, which were collected by filtration to yield the titled compound (12.2 g).
m.p. 92.2 - 94.1°C
IR Spectrum (KBr; cm⁻¹)
   3269, 1741, 1344, 1255, 1151, 608
NMR Spectrum (DMSO-d₆; ppm)*
   2.36 (3H, s)
   2.53 (3H, s)
   3.55 (2H, d, J=5.9 Hz)
   6.87 (1H, s)
   7.86 (1H, t, J=5.9 Hz)

### 〈Step 3〉 Synthesis of 1-(2,5-dimethylthien-3-ylsulfonyl)thiohydantoin

To the compound (11 g) obtained in step 2, ammonium thiocyanate (10.1 g), anhydrous pyridine (10.2 mL) and acetic anhydride (21 mL) were added and the mixture was thereafter stirred at 70°C for 3 h. After cooling, the reaction solution was poured into 1 N HCl (100 mL) and the resulting precipitate was collected by filtration, washed with a small amount of ethanol and dried with air to yield the titled compound (6.3 g).
m.p. 208°C (with decomposition)
IR Spectrum (KBr; cm⁻¹)
   3396, 1750, 1456, 1367, 1155, 1084
NMR Spectrum (DMSO-d₆; ppm)*
   2.37 (3H, s)
   2.65 (3H, s)
   4.71 (2H, s)
   7.17 (1H, s)
   12.57 (1H, bs)

### 〈Step 4〉 Synthesis of 1-(2,5-dimethylthien-3-ylsulfonyl)hydantoin

The compound (5 g) obtained in step 3 was treated by the same method as step 4 in Example 1 to yield the titled compound (1.8 g). The analytical data are shown in Table 2 under "Compound 4".

### Example 5 Synthesis of 1-(indan-2-ylsulfonyl)hydantoin (Compound 5)

### 〈Step 1〉 Synthesis of 2-acetylthioindane

Indene (100 mL) was treated by the same method as step 1 in Example 3 to yield the titled compound (148 g).
m.p. 41.9 - 44.6°C
IR Spectrum (KBr; cm⁻¹)
   1684, 1462, 1128, 1097, 953, 914, 744, 640
NMR Spectrum (CDCl₃; ppm)*
   2.32 (3H, s)
   2.90 (2H, dd, J=6.1, 16.0 Hz)
   3.45 (2H, dd, J=7.4, 16.0 Hz)
   4.24 (1H, tt, J=6.1, 7.4 Hz)
   7.13 - 7.26 (4H, m)

### 〈Step 2〉 Synthesis of N-(indan-2-ylsulfonyl)glycine

The compound (20 g) obtained in step 1 was treated by the same method as step 2 in Example 2 to yield the titled compound (12 g).
m.p. 140.4°C (with decomposition)
IR Spectrum (KBr; cm⁻¹)
   3336, 2956, 1720, 1425, 1317, 1238, 1134
NMR Spectrum (CDCl₃; ppm)*
   3.12 - 3.90 (4H, m)
   3.93 (2H, d, J=5.3 Hz)
   3.95 - 4.32 (1H, m)
   4.97 (1H, t, J=5.3 Hz)
   7.20 - 7.38 (4H, m)

### 〈Step 3〉 Synthesis of 1-(indan-2-ylsulfonyl)thiohydantoin

The compound (11 g) obtained in step 2 was treated by the same method as step 3 in Example 2 to yield the titled compound (5.4 g).
m.p. 182.6°C (with decomposition)
IR Spectrum (KBr; cm⁻¹)
   3138, 1790, 1761, 1462, 1425, 1360, 1236
NMR Spectrum (CDCl₃; ppm)*
   3.18 - 3.78 (4H, m)
   4.52 (2H, s)
   5.32 (1H, quint, J=8.9 Hz)
   7.08 - 7.26 (4H, m)

### 〈Step 4〉 Synthesis of 1-(indan-2-ylsulfonyl)hydantoin

The compound (5.3 g) obtained in step 3 was treated by the same method as step 4 in Example 1 to yield the titled compound (2.8 g). The analytical data are shown in Table 2 under "Compound 5".

### Example 6 Synthesis of 1-(2,5-dichlorophenylsulfonyl)hydantoin (Compound 6)

### 〈Step 1〉 Synthesis of N-(2,5-dichlorophenylsulfonyl)glycine

Anhydrous potassium carbonate (17 g) was dissolved in 50 mL of purified water and glycine (9.3 g) was added to dissolve. To the solution, 2,5-dichlorophenylsulfonyl chloride (25 g) was added and after heating at 40 - 50°C for 10 min., the mixture was further heated in a boiling water bath for 1 h. After cooling, it was acidic (pH 2 - 3) with 2N HCl and the resulting precipitate was collected by filtration to yield the titled compound (28.1 g).

### 〈Step 2〉 Synthesis of 1-(2,5-dichlorophenylsulfonyl)thiohydantoin

To the compound (11.5 g) obtained in step 1, anhydrous pyridine (3.3 mL), dried ammonium thiocyanate (6.1 g) and acetic anhydride (8.2 mL) were added and the mixture was heated with stirring in a boiling water bath for 30 min. After leaving the reaction system to cool, 80 mL of purified water was added and the mixture was cooled with ice; the resulting precipitate was collected by filtration to yield the titled compound (7.4 g).

### 〈Step 3〉 Synthesis of 1-(2,5-dichlorophenylsulfonyl)hydantoin

To the compound (13.3 g) obtained in step 2, 100 mL of 50% (vol/vol) nitric acid was added and the mixture was heated in a boiling water bath for 90 min.; upon cooling with ice, the resulting precipitate was collected by filtration to yield the titled compound (5.1 g). The analytical data are shown in Table 2 under "Compound 6".

The foregoing analytical data were obtained as follows: NMR measurements were made with JEOL FX90A FT-NMR (data asterisked; manufactured by JEOL LTD.) or JEOL JNM-EX270 FT-NMR (manufactured by JEOL LTD.); IR measurements were made with HORIBA FT-200 (manufactured by HORIBA, LTD.); and m.p. measurements were made with Mettler FP80 or FP90 (both manufactured by Mettler Instruments AG). (Formulation Examples)

The present invention will now be described with reference to the following formulation examples but it is in no way limited thereto.

### Example A Tablet

| | |
|---|---|
| Compound 2 | 125 g |
| Lactose | 725 g |
| Cornstarch | 120 g |
| Polyvinyl alcohol | 15 g |
| Magnesium stearate | 15 g |

After weighing the ingredients listed above, compound 2, lactose and cornstarch were mixed uniformly and, following the addition of an aqueous solution of polyvinyl alcohol, the mixture was processed by a wet granulation method to prepare granules. The granules were dried, mixed with magnesium stearate and, thereafter, was made into tablets each weighing 200 mg by compression tabletting.

### Example B Tablet

| | |
|---|---|
| Compound 1 | 300 g |
| Lactose | 550 g |
| Cornstarch | 120 g |
| Polyvinyl alcohol | 15 g |
| Magnesium stearate | 15 g |

After weighing the ingredients listed above, the same method was employed as in Example A to make tablets each weighing 200 mg.

### Example C Granule

| | |
|---|---|
| Compound 4 | 200 g |
| Lactose | 150 g |
| Cornstarch | 400 g |
| Crystalline cellulose | 200 g |
| Hydroxypropyl cellulose | 50 g |

After weighing the ingredients listed above, compound 4, lactose, cornstarch and crystalline cellulose were mixed uniformly and, following the addition of an aqueous solution of hydroxypropyl cellulose, the mixture was processed by a wet granulation method to prepare granules. The granules were dried and classified.

### Example D Capsule

| | |
|---|---|
| Compound 5 | 500 g |
| Lactose | 480 g |
| Magnesium stearate | 20 g |

The ingredients listed above were weighed and mixed uniformly. The powder mixture was filled into No. 1 hard capsules in amounts of 200 mg.

### Example E Suppository

| | |
|---|---|
| Compound 3 | 300 g |
| Polyethylene glycol 4000 | 600 g |
| Polyethylene glycol 1500 | 100 g |

Compound 3 was milled thoroughly in a mortar to form a fine powder, which was processed by a melt molding method to prepare rectal suppositories each weighing 1 g.

### Example F Powder

| | |
|---|---|
| Compound 6 | 200 g |
| Lactose | 790 g |
| Magnesium stearate | 10 g |

The ingredients listed above were weighed and mixed uniformly to form a 20% powder.

### INDUSTRIAL APPLICABILITY

The uricosuric agents of the invention are drugs that exhibit a potent uricosuric action in laboratory animal models while maintaining or mildly increasing the volume of urine; at the same time, they have low toxicity and hence are medicines useful in the treatment of hyperuricemia or in the treatment and prevention of gout. Because of their ability to maintain or mildly increase the urine volume, the uricosuric agents of the invention are anticipated to relieve patients of the burden of taking much water.

## Claims

1. A therapeutic agent of hyperuricemia characterized by containing a hydantoin derivative of the general formula (I): 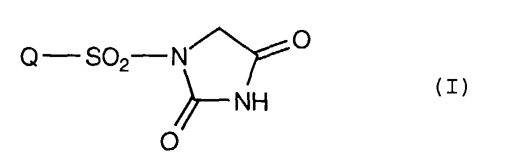 (where Q denotes a cyclohexyl group, a cycloheptyl group, a cyclooctyl group, a 2,5-dimethylthien-3-yl group, an indan-2-yl group or a 2,5-dichlorophenyl group) or a salt thereof as an active ingredient.

2. An agent for preventing or treating gout which is characterized by containing a hydantoin derivative of the general formula (I) (where Q has the same definition as given in claim 1) or a salt thereof as an active ingredient.

3. A uricosuric agent characterized by containing a hydantoin derivative of the general formula (I) (where Q has the same definition as given in claim 1) or a salt thereof as an active ingredient.

4. A hydantoin derivative of the general formula (I'): 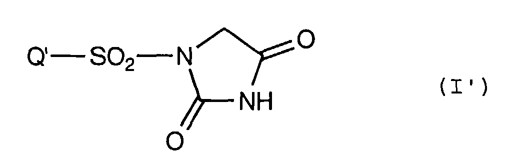 (where Q' denotes a cycloheptyl group, a cyclooctyl group, a 2,5-dimethylthien-3-yl group or an indan-2-yl group) or a salt thereof.

5. The use of a hydantoin derivative of the general formula (I) (where Q has the same definition as given in claim 1) or a salt thereof for the production of drugs as therapeutics of hyperuricemia, preventives or therapeutics of gout and/or uricosuric agents.

6. A method of treating hyperuricemia and/or a method of preventing or treating gout by an effective amount of a hydantoin derivative of the general formula (I) (where Q has the same definition as given in claim 1) or a salt thereof.

7. A method of excreting uric acid by an effective amount of a hydantoin derivative of the general formula (I) (where Q has the same definition as given in claim 1) or a salt thereof.
